# NEW EUROPEAN PATENT SPECIFICATION

(11) **EP 0 511 308 B2**
(45) Date of publication and mention of the opposition decision: **09.07.2003**
(45) Mention of the grant of the patent: 25.09.1996
(21) Application number: 91903980.0
(22) Date of filing: 27.12.1990
(51) Int. Cl.: C07K 16/46, C12N 15/62, C12N 15/13, C12N 15/85, A61K 39/395

(54) **CHIMERIC IMMUNOGLOBULIN FOR CD4 RECEPTORS**
SCHIMÄRE IMMUNOGLOBULINE FÜR CD4-REZEPTOREN
IMMUNOGLOBULINE CHIMERIQUE POUR RECEPTEURS DE CD4

(30) Priority: 27.12.1989 US 457389
(43) Date of publication of application: 04.11.1992
(73) Proprietor: CENTOCOR, INC., Malvern, PA 19355 (US)
(72) Inventor: GHRAYEB, John, Thorndale, PA 19372 (US); KNIGHT, David, M., Paoli, PA 19301 (US); LOONEY, James, E., Timonium, MD 21903 (US)
(74) Representative: Kirkham, Nicholas Andrew
(86) International application number: US9007671
(87) International publication number: WO91010722

(56) References cited:
- EP-A- 0 125 023
- EP-A- 0 240 344
- EP-A- 0 365 209
- EP-A- 0 394 827
- WO-A-88/09181
- WO-A-90/12868
- Immunobiology, vol. 181, pp. 219-220, 1990
- Letters to Nature, vol.339, 4 May 1989, A.Traunecker et al: "Highly efficient neutralization of HIV with recombinant CD4-immunoglobulin molecules" pages 68-70
- Oi and Morrison (1986) Immunobiology 181 (2/3) 219
- Herzog et al (1989) J Autoimmunity 2, 627-642
- Kong et al (1989) Clin.Exp.Immunol 77, 428-433
- Sattenau et al (1986) Science 234, 1120-1123

## Description

### Background of the invention

The nature of autoantigens responsible for autoimmune disorders is not known, nor is the action which triggers the autoimmune response. One theory involves the similarity of a viral protein to a self antigen, which results in auto-reactive T cells or B cells recognizing a self antigen. Whereas B-lymphocytes produce antibodies, thymus-derived or "T-cells" are associated with cell-mediated immune functions. T-cells recognize antigens presented on the surface of cells and carry out their functions with these "antigen-presenting" cells.

Various markers have been used to define human T cell populations. CD4 is a non-polymorphic surface glycoprotein receptor with partial sequence identity to immunoglobulins. CD4 receptors define distinct subsets of mature peripheral T cells. In general, CD4 T cells express helper or regulatory functions with B cells in immune responses, while T cells express the CD8 surface antigen function as cytotoxic T cells and have suppressive effects in immune responses. The CD4 receptor consists of a signal peptide, a 370 amino acid extracellular region containing four tandem immunoglobulin-like domains (V₁-V₄), a membrane spanning domain, and a charged, intracellular region of forty (40) residues.

Since T-cell receptors are thought to augment or modulate T-cell response, they present a potential target for immunological intervention. One approach to the treatment of autoimmune disorders involves monoclonal antibodies specific for CD4 receptors. Murine anti-CD4 monoclonal antibodies appear useful in the treatment of rheumatoid arthritis as disclosed in Hertzog, C. et al. Lancet, page 1461 (December 19, 1987). Murine antibodies, nowever, have characteristics which may severely limit their use in human therapy. As foreign proteins, murine antibodies may elicit immune reactions that reduce or destroy their therapeutic efficacy and/or evoke allergic or hypersensitivity reaction in patients. The need for readministration of such therapeutic modalities in autoimmune disorders increases the likelihood of these types of immune reactions.

Chimeric antibodies consisting of non-human antigen binding regions joined to human constant regions have been suggested as a means to circumvent the immunogenicity of murine antibodies See e.g. PNAS, 81:6851 (1984) and WO-A-86/01533. Since the constant region is largely responsible for immunogenicity of an antibody molecule, chimeric antibodies with constant regions of human origin should be less likely to evoke an anti-murine response in humans. However, it is unpredictable whether the joining of a human constant region to a nonhuman antigen binding region of a desired specificity will reduce immunoreactivity and/or alter the binding capabilities or the biological activity of the resulting chimeric antibody. Furthermore, immunoglobulin constant regions exist as a variety of isotypes, which are responsible for different effector functions. Therefore the biological activity of chimeric antibody will depend on the isotype of the constant regions as well as the nature of the antigen-binding regions.

Not all anti-CD4 monoclonal antibodies bind to the same CD4 site or domain, and the site to which a particular monoclonal antibody binds may significantly affect its biological activity, e.g., its immunomodulatory activity, or its ability to block the binding of HIV to CD4 cells, for example.

WO 92/05274, WO 91/09966 and Reiter et al (1990), immunobiology, 181: 219-220, Abstract NO. J.8 disclose humanized monoclonal antibodies, but do not disclose, suggest and/or enable the chimeric immunoglobulins of the claims.

### Summary of the Invention

This invention pertains to chimeric immunoglobulins specific for a CD4 receptor and able to: (1) induce sustained depletion of CD4⁺T cells in humans, and (2) compete with the M-T151 mAb for binding to CEM cells. The immunoglobulin of the invention comprises a variable or antigen binding region of a non-human origin specific for CD4 receptor and a constant region of human origin. The invention also covers pharmaceutical compositions containing the immunoglobulins and their use for the manufacture of medicaments for the treatment of diseases and disorders mediated by CD4 positive cells. These antibodies are useful as therapeutic agents for autoimmune disorders, and other diseases or disorders mediated by CD4 positive cells.

### Brief Description of the Figure

The figure shows the plasmids for expression of the chimeric chains of the chimeric anti-CD4 antibody.

### Detailed Description of the Invention

The present invention relates to a method for producing a CD4-specific chimeric immunoglobulin comprising an antigen binding region of non-human origin and a constant region of human origin the chimeric immunoglobulin being:
(1) capable of inducing sustained depletion of CD4⁺ T cells in humans, and (2) able to compete with the M-T151 mAb for binding to CEM cells.

In another aspect, the invention relates to a chimeric immunoglobulin comprising:
a. at least one chimeric heavy chain comprising an antigen binding region derived from the heavy chain of a non-human immunoglobulin specific for CD4 receptor linked to at least a portion of a human heavy chain constant region, the heavy chain being in association with:
b. at least one chimeric light chain comprising an antigen binding region derived from a light chain of the non-human immunoglobulin linked to at least a portion of a human light chain constant region, the chimeric immunoglobulin being: (1) capable of inducing sustained depletion of CD4⁺ T cells in humans, and (2) able to compete with the M-T151 mAb for binding to CEM cells.

The human heavy chain constant region is preferably of the IgGI isotype. The-chimeric light chain comprises an antigen binding region derived from the light chain of the non-human antibody linked to a human light chain constant region. The human light chain constant region is preferably of the Kappa isotype.

The present immunoglobulins can be monovalent, divalent or polyvalent. Monovalent immunoglobulins are dimers (HL) formed of a chimeric heavy chain associated through disulfide bridges with a chimeric light chain. Divalent immunoglobulins are tetrameres (H₂L₂) formed of two dimers associated through at least one disulfide bridge. Such divalent immunoglobulins are preferred for biological activity. Polyvalent immunoglobulins can also be produced, for example, by employing heavy chain constant regions that aggregate (e.g. IgM heavy chains). Chimeric immunoglobulin fragments such as Fab, Fab', or F(ab')₂ can also be produced, and may be particularly useful for some applications. The non-human antigen binding regions of the chimeric immunoglobulin are derived from immunoglobulins specific for CD4 receptors. Preferred for biological activity are antibodies of the invention that exhibit high affinity binding to a CD4 site encompassing residues within both the V₁ and the V₂ domains. Preferred are antibodies of the invention that exhibit high affinity binding to CD4, preferably a Ka of at least 10⁸M⁻¹, more preferably at least 10⁹M⁻¹.

The chimeric anti-CD4 monoclonal antibodies of the invention will preferably bind specifically (with high affinity) to a site encompassing residues in both the extracellular V₁ and V₂ domains, a site distinct from the Leu 3a site and the OKT4 site.

A preferred chimeric murine-human MAb of the invention, designated cM-T412, and murine antibody M-T151 disclosed in J. of Autommunity 2, 627-642 (1989) are believed to be directed to substantially the same CD4 binding regions. The binding specificity of M-T151 for CD4 has been mapped to an epitope encompassing residues in both the extracellular V₁ and V₂ domains of the CD4 protein by crossblocking analyses and specific binding to truncated recombinant CD4. Peterson et al., Cell 54:65-72 (1988); Ashkenzi et al., Proc. Natl. Acad. Sci. USA 87:7150-7156 (1990); Healey et al., J. Ex. Med. 172:1233-1242 (Oct. 1990) and Sattentau et al., J. Ex. Med. 170:1319-1334 (Oct. 1989; Ryu et al., Nature 348:419-426 (1990) and Wang et al. Nature 348:411-418 (1990) Mapping of anti-CD4 monoclonal antibodies to the CD4 receptor is complicated by the fact that many important CD4 epitopes appear to be nonlinear, conformational epitopes. Much information about CD4 epitopes comes from cross blocking studies and other analytical procedures as described in the foregoing references, the teachings of which are hereby incorporated herein by reference.

A competition study was carried out to compare CD4 binding of murine M-T412 and murine M-T151. The experiment was performed two ways: (1) 125I M-T412 competing with increasing concentrations of unlabeled M-T412 and M-T151; and (2) 1261 M-T151 competing with increased concentrations of unlabeled M-T412 and M-T151. Results indicated that M-T151 and M-T412 cross comeete for binding to CEM cells to the same extent and with similar binding kinetics. This suggests that the most efficient inhibition results from direct competition for the same or adjacent epitopes. Sattentau et al., Science 232:1120-1123 (1986) Preliminary cross blocking data with a panel of anti-CD4 monoclonal antibodies suggest that the epitopes to which M-T412 and M-T151 are directed are substantially the same but probably not identical. Accordingly, a preferred chimeric murine-human anti-CD4 antibody of the invention is one which comprises murine variable regions substantially similar to those of murine clones M-T412 or M-T151, most preferably an intact divalent immunoglobulin which also comprises a human Fc regions of the IgG1 isotype.

The present cM-T412 chimeric anti-CD4 monoclonal antibody is further preferred as being directed to a conserved CD4 epitope. A binding study of cM-T412 with peripheral blood from 50 normal donors representing both sexes and an ethnic mix, suggested that cM-T412 binds to a conserved epitope, since all samples exhibited the same magnitude and kinetics of immunoreactivity.

Preferred chimeric anti-CD4 monoclonal antibodies of the invention are those which will competitively inhibit the binding to CD4 receptor of a chimeric anti-CD4 monoclonal antibody substantially similar to cM-T412. Quantities of the cM-T412 antibody, designated "c128", are deposited as a reference standard at Centocor, Malvern, PA, USA, and at the American Type Culture Collection (ATCC), Rockville, MD, USA on December 21, 1990 and received ATCC No. 40942. cM-T412 (c128) is a chimeric monoclonal antibody produced by cell line C128A, which is on deposit as a master cell bank in the Centocor Cell Culture Research & Development Depository, Malvern, PA, USA, and at Centocor BV, Leiden, The Netherlands. The cM-T412 chimeric murine-human monoclonal antibody is a specifically preferred embodiment of the invention. The teaching of Muller, R. (1983), "Determination of affinity and specificity of anti-hapten antibodies by competitive radioimmunoassay", Methods in Enzymology U92:589-601, with respect to methods for determining competitive inhibition of monoclonal antibody binding, is hereby incorporated herein by reference.

Preferred immunoglobulins are produced by antibody-producing cell lines which may be hybrid cell lines commonly known as hybridomas. The hybrid cells are formed by the fusion of an anti-CD4 antibody producing cell and an immortalizing cell line, that is, a cell line which imparts long term tissue culture stability to the hybrid cell. In the formation of the hybrid cell lines, the first fusion partner - the anti-CD4 antibody producing cell - may be a spleen cell of an animal immunized against a CD4 positive T cell or a biological preparation comprising CD4. Alternatively, the anti-CD4 producing cell may be a B lymphocyte obtained from the spleen, lymph nodes or other tissue. The second fusion partner - the immortal cell - may be a lymphoblastoid cell or a plasmacytoma cell such as a myeloma cell, itself an antibody producing cell, but also maligant.

Murine hybridomas which produce CD4 specific monoclonal antibodies are formed by the fusion of mouse myeloma cells and spleen cells from mice immunized against human CD4 positive T cells, purified CD4, or other biological preparations comprising CD4, or a component thereof. For example, a preparation comprising P815 mastocytoma cells transfected with human CD4 cDNA was used successfuly in the Examples. To immunize the mice, a variety of different protocols may be followed. For example, mice may receive primary and boosting immunizations of CD4 positive T cells or recombinant CD4. The fusions are accomplished by standard procedures well known to those skilled in the field of immunology. Kohler and Milstein, Nature, 256:495-497 (1975) and Kennet, Monoclonal Antibodies (Kennet et al., Eds. pp. 365-367, Plenum Press, N.Y, 1980). Several murine CD4 specific monoclonal antibodies are described in Herzog, C. et al., supra; Herzog, C. et al. J. Autoimmunity 2:627-642 (1989) and Walker et al. J. Autoimmunity 2: 643-649 (1989).

Another way of forming the anti-CD4 producing cell line is by transformation of antibody producing cells. For example, an anti-CD4 producing B lymphocyte may be infected and transformed with a virus such as Epstein-Barr virus in the case of B lymphocytes to yield an immortal anti-CD4 producing cell. See e.g., Kozbor and Roder, Immunology Today, 4(3):72-79 (1983). Alternatively, the B lymphocyte may be transformed by a transforming gene or transforming gene product.

The CD4 specific monoclonal antibodies are produced in large quantities by injecting anti-CD4 antibody producing hybridomas into the peritoneal cavity of mice and, after appropriate time, harvesting the ascites fluid which contains a high titer of homogeneous antibody and isolating the monoclonal anti-CD4 antibody therefrom. Xenogeneic hybridomas should be injected into irradiated or athymic nude mice. Alternatively, the antibodies may be produced by culturing anti-CD4 producing cells in vitro and isolating secreted monoclonal anti-CD4 antibodies from the cell culture medium.

The CD4 specific chimeric antibodies of the invention are produced by cloning DNA segments encoding the heavy and light chain variable regions of a non-human antibody specific for CD4 and joining these DNA segments to respective DNA segments encoding human heavy and light chain constant regions to produce chimeric immunoglobulin encoding genes. The fused gene constructs coding for the light and heavy chains are assembled in or inserted into expression vectors. The genes are co-transfected into a lymphoid recipient cell (e.g. a myeloma cell) where the immunoglobulin protein can be synthesized, assembled and secreted. The transfected receipient cells are cultured and the expressed immunoglobulins are collected.

Preferably, the antigen binding regions will be of murine origin because murine antibodies against CD4 are available or can be readily produced in murine systems. Other animal or rodent species provide alternative sources of antigen binding regions.

The constant regions of the chimeric antibodies are derived from human immunoglobulins. The heavy chain constant region can be selected from any of the five isotypes - alpha, delta, epsilon, gamma or mu. Further, heavy chains of various subclasses (such as the IgG subclasses of heavy chains) are responsible for different effector functions and thus, by choosing the desired heavy chain constant region, chimeric antibodies with desired effector function can be produced. Preferred constant regions are gamma 1 (IgG1), gamma 3 (IgG3) and gamma 4 (IgG4). More preferred is an Fc region of the gamma 1 (IgG1) isotype. The light chain constant region can be of the kappa or lambda type, preferably of the kappa type.

In order to assess the effect of human heavy chain constant region isotype on the activity of chimeric anti-CD4 antibodies of the invention, mouse-human chimeric CD4 IgG1 and IgG4 antibodies were constructed with murine M-T412 variable regions and human Fc constant regions. F(ab')₂ and Fab fragments of the murine (M-T412) and chimeric γ1 (cM-T412) antibodies were generated by enzymatic digestion. The cM-T412γ1 and its fragments retained the affinity and specificity of the parent murine antibody.

The ability of intact CD4 antibody or antibody fragment to affect CD4⁺ T-cell activity was evaluated in in vitro assays of Ig production by pokeweed mitogen-stimulated cells, level of slL-2 receptor production by phytohemagglutinin-stimulated PBMCs (peripheral blood mononuclear cells), and cell proliferation in response to: tetanus toxoid, anti-CD3 antibodies, and mixed lymphocyte culture. Representative findings are seen with tetanus toxoid where the CM-T412γ1 MAb, the intact divalent (H₂L₂) immunoglobulin of the γ1 isotype, inhibited the proliferation of PBMCs by 90% at 0.1µ/ml. In contrast, the cM-T412γ4 achieved a maximum of 65% inhibition even at 10 µg/ml, whereas the cM-T412γ1 Fab required 100 µg/ml for similar inhibition. These data show that the intact chimeric mouse-human anti-CD4γ1 antibody of the invention exhibits superior down regulation of T-cell function, with a strong contribution by the γ1 F_{c} region. These results support the potential clinical utility of a divalent chimeric mouse-human anti-CD4 γ1 monoclonal antibody in autoimmune disease or disorders.

In general, the chimeric antibodies are produced by preparing, for each of the light and heavy chain components of the chimeric immunoglobulin, a fused gene comprising a first DNA segment that encodes at least the functional portion of the CD4-specific variable region of non-human origin linked (e.g. functionally rearranged variable region with joining segment) to a second DNA segment encoding at least a biologically functional part of a human constant region. Each fused gene is assembled in or inserted into an expression vector. Recipient cells capable of expressing the gene products are then transfected with the genes. The transfected recipient cells are cultured under conditions that permit expression of the incorporated genes and the expressed immunoglobulins or immunoglobulin chains are recovered.

Genes encoding the variable region of Ig light and heavy chains can be obtained from lymphoid cells that produce the CD4-specific antibodies. For example, the hybridoma cell lines that produce antibody against CD4 provide a source of immunoglobulin variable region for the present chimeric antibodies. Other rodent cell lines are available. Cell lines can be produced by challenging a rodent with a CD4-positive cell or a CD4 containing component or fraction of a CD4 positive cell, forming fused hybrid cells between antibody-producing cells and a myeloma cell line, cloning the hybrid and selecting clones that produce antibody against the CD4 receptor. Antibodies can be further characterized as to epitope specificity (high affinity binding), as described in Sattentau et al., supra, the teachings of which are hereby incorporated by reference.

It is contemplated that further "humanization" of the monoclonal antibodies of the invention may be accomplished by forming "mosaic" antibodies in which human sequences are also inserted into the variable region. For example, the variable regions of both mouse and human antibodies comprise four framework residues (FRs). Within the FRs are three complementarity determining residues (CDRs) which are responsible for antigen binding. A human-mouse mosaic having the desired binding characteristics may be made by inserting mouse CDR sequences within human framework residues. Such mosaic variants are contemplated equivalents of the chimeric immunoglobulins of the invention, as are partial chimeric immunoglobulins, e.g., in which only the heavy chain constant region of murine origin has been replaced by an equivalent sequence of human origin, or variants wherein one or more amino acids have been changed by directed mutagenesis.

Constant regions can be obtained from human antibody-producing cells by standard cloning techniques. Alternatively, because genes representing the two (2) classes of light chains and the five (5) classes of heavy chains have been cloned, constant regions of human origin are readily available from these clones. Chimeric antibody binding fragments such as F(ab')₂ and Fab fragments can be prepared by designing a chimeric heavy chain gene in truncated form. For example, a chimeric gene encoding a F(ab')₂ heavy chain portion would include DNA sequences encoding the CH₁ domain and hinge region of the heavy chain.

The fused genes encoding the light and heavy chimeric chains (or portions therof) can be assembled in two different expression vectors that can be used to co-transfect a recipient cell. Each vector contains two (2) selectable genes -- one for selection in a bacterial system and one for selection in a eukaryotic system - each vector having a different pair of genes. These vectors allow production and amplification of the fused genes in bacterial systems, and subsequent co-transfection of eukaryotic cells and selection of the co-transfected cells. Examples of selectable genes for the bacterial system are the genes that confer ampicillin resistance and the gene that confers chloramphenicol resistance. Two selectable genes for selection of eukaryotoic transfectants are preferred: (i) the xanthine-guanine phosphoribosyltransferase gene (gpt), and (ii) the phosphotransferase gene from Tn5 (designated neo). Selection with gpt is based on the ability of the enzyme encoded by this gene to use xanthine as a substrate for purine nucleotide synthesis; the analogous endogenous enzyme cannot. In a medium containing xanthine and mycophenolic acid, which blocks the conversion of inosine monophosphate to xanthine monophosphate, only cells expressing the gpt gene can survive. The product of the neo blocks the inhibition of protein synthesis in eukarytoic cells caused by the antibiotic G418 and other antibiotics of its class. The two selection procedures can be used simultaneously or sequentially to select for the expression of immunoglobulin chain genes introduced on two (2) different DNA vectors into a eukaryotic cell.

Alternatively, the fused genes encoding the chimeric light and heavy chains can be assembled on the same expression vector.

The preferred recipient cell line is a myeloma cell. Myeloma cells can synthesize, assemble and secrete immunoglobulins encoded by transfected Ig genes. Further, they possess the mechanism for glycosylation of the immunoglobulin. A particularly preferred recipient cell is the Ig-non-producing myeloma cell Sp2/0. The cell produces only immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Several methods exist for transfecting lymphoid cells with vectors containing immunoglobulin encoding genes. A preferred way of introducing DNA into lymphoid cells is by electroporation. In this procedure, recipient cells are subjected to an electric pulse in the presence of the DNA to be incorporated. See e.g., Potter et al., PNAS 81:7161 (1984). Another way to introduce DNA is by protoplast fusion. In this method, lysozyme is used to strip cell walls from bacteria harboring the recombinant plasmid containing the chimeric Ig gene. The resulting spheroplasts are fused with myeloma cells with polyethylene glycol. Another technique that can be used to introduce DNA into many cell types is calcium phosphate precipitation.

The chimeric immunoglobulin genes can be expressed in nonlymphoid mammalian cells or in organisms such as bacteria or yeast. When expressed in bacteria, the immunoglobulin heavy chains and light chains become part of inclusion bodies. Thus, the chains must be isolated and purified and then assembled into functional immunoglobulin molecules.

The chimeric CD4 specific antibodies of the invention are useful as therapeutic agents for autoimmune disorders such as rheumatoid arthritis, SLE, multiple sclerosis and myasthenia gravis, as well as other disorders mediated by CD4+ cells. The antibody is administered to a mammal suffering from such a disorder in a therapeutically effective amount sufficient to alleviate the disorder.

The chimeric monoclonal antibodies of the invention will usually be formulated for therapeutic use as a pharmaceutical composition comprising appropriate carriers, excipients, and other pharmaceutically acceptable ingredients, as is known to those of skill in the art of pharmaceutics. Like other proteinaceous materials, a monoclonal antibody preparation will frequently be formulated as a sterile, non-pyrogenic composition for parenteral administration; however, any pharmaceutically acceptable route and method of administration that brings that active moiety into contact with its site of action may be used, such as those described in a standard reference text in this field, e.g., Remington's Pharmaceutical Sciences, the teachings of which are hereby incorporated by reference.

The invention is further described by the following examples, wherein all parts and percentages are by weight and degrees are Celsius, unless otherwise stated.

### EXEMPLIFICATION

### Murine Hybridoma M-T412

A murine hybridoma designated M-T412 was one of a panel of murine anti-CD4 monoclonal antibodies obtained from G. Riethmuller, Univ. of Munich, Munich, Germany. The M-T412 clone was produced from spleen cells of mice immunized with P815 mastocytoma cells transfected with human CD4 cDNA. The mice were additionally boosted with recombinant CD4. The M-T412 antibody was of high affinity for CD4 receptor bearing CEM cells and of similar binding specificity to a murine anti-CD4 monoclonal antibody designated MT151 (described in papers by Herzog et al. and Walker et al., supra.) The details of the preparation of murine hybridoma M-T412 are as follows: The hybridoma giving rise to the murine M-T412 antibody was derived from a fusion performed in the laboratory of Dr. Peter Rieber, Institute for Immunologie, Universitat Munchen, Munich, Germany.

BALB/c mice originally obtained from the Centralinstitut fur Versuchstierzucht (Hanover, Germany) and bred at the Institute for Immunologie, Munich, Germany were immunized with P815 cells (obtained from Van Pel, Ludwig Institute for Cancer Research, Brussels, Belgium) transfected with cloned DNA representing a full length copy of the gene encoding human CD4. The CD4 clone was derived from a cDNA library provided by D. Littman (University of California, San Francisco, CA, USA). The cDNA was cleaved with Xho II, cloned in pKSv10 and the fragment representing the full length CD4 gene was transfected into P815 cells by Y. Tabaycewski and E. Weiss, Institute for Immunologie, Munich.

A BALB/c mouse was immunized intrasplenically with 5 x 10⁶ P815-CD4 cells. Forty days later the mouse was injected again intrasplenically with 5 x 10⁶ P815-CD4 cells. Three days later the mouse was sacrificed, the spleen was removed and a single cell suspension was obtained by mechanical disaggregation. The spleen cells were resuspended in RPMI 1640 containing 20% (v/v) fetal bovine serum and 10% (v/v) DMSO and cryopreserved in liquid nitrogen.

The cells were subsequently thawed and 3.2 x 10⁷ spleen cells were fused with 2.4 x 10⁷ P3x63Ag8.653 (.653) myeloma cells (provided by H. Lemke, Cologne, Germany). The spleen cells and 653 cells were mixed and centrifuged for 10 min at 1600 rpm. The supernatant was removed and the pellet was resuspended in 5 mL prewarmed, serum free DMEM, centrifuged at 1200 rpm, for 5 min. The supernatant was removed, and 1 ml 50% (w/v) PEG 4000 in RPMI 1640 (prewarmed to 37°C) was added dropwise. The cells were then centrifuged for 1 min at 1000 rpm followed by addition of 10 mL RPMI 1640 (37°C) without serum dropwise over 5 min. The pelle was resuspended, another 10 ml of medium added and the sample centrifuged fro 10 min at 1600 rpm. The supernatant was removed and the cells were resuspended in HAT medium (RPMI 1640 supplemented with 10% (v/v) fetal bovine serum; 100 µ/mL penicillin; 100 µg/mL streptomycin; 6.4 x 10⁻⁵ M thymidine. The fused cells were seeded at 100 µL/well into each well of 96-well plates. Fifty thousand (50,000) BALB/c-DBA fl peritoneal exudate cells in 100 µL were added to each well. The cells were fed with HT medium (HAT with aminopterin) after 4 and 7 days and assayed on day 10-14.

Four-hundred and fifty (450) growth positive wells were screened against normal human peripheral blood mononuclear cells by fluorescence microscopy. From this fusion, 3 anti-CD4 hybridomas were obtained and designated M-T412, M-T413, and M-T414.

The M-T412 cell line was grown in Iscove's modification of Dulbecco's modified Eagle's Minimum Essential Medium (IDMEM supplemented with 5% (v/v) fetal bovine serum, 2 mM L-glutamine, 1 mM sodium pyruvate and Eagle's Non-Essential Amino Acids). A cell bank created from this culture was used for production of the chimeric anti-CD4 monoclonal antibody.

### General Strategy for Production of Chimeric Anti-CD4 Monoclonal Antibodies

The strategy for cloning the variable regions for the heavy and light chain genes from the hybridoma M-T412 was based upon the linkage in the genome between the variable region and the corresponding J (joining) region for functionally rearranged (and expressed) Ig genes. J region DNA probes can be used to screen genomic libraries to isolate DNA linked to the J regions; DNA in the germline configuration (unrearranged) would also hybridize to J probes, but is not linked to a varible region sequence and can be indentified by restriction enzyme analysis of the isolated clones.

The cloning strategy, therefore, was to isolate variable regions from rearranged heavy and light chain genes using J_{H} and J_{K} probes. These clones were tested to see if their sequences were expressed in the 412 hybridoma by Northern analysis. Those clones that contained expressed sequence were cloned into expression vectors containing human constant regions and transfected into mouse myeloma cells to determine if an antibody was produced. The antibody from producing cells was then tested for binding specificity and functionality compared to the 412 murine antibody.

### Materials and Methods

### Light chain genomic library construction

To isolate the light chain variable region gene from the 412 hybridoma, a size-selected genomic library was constructed using the phage lambda vector charon 27. High molecular weight DNA was isolated from 412 hybridoma cells and digested to completion with restriction endonuclease HindIII. The DNA was then fractionated on a 0.8% agarose gel and the DNA of size range 4-6 kb was isolated directly from the gel by electroelution. After phenol/chloroform extraction and ethanol precipitation, the 4-6 kb fragments were ligated with lambda charon 27 arms and packaged into phage particles in vitro using Gigapack Gold from Stratagene. This library was screened directly at a density of approximately 20,000 plaques per 150 mm petri dish using a ³²P-labeled J_{K} probe. Plaque hybridizations were carried out in 5x SSC, 50% formamide, 2x Denhardt's reagent, 200 µg/ml denatured salmon sperm DNA at 42 degrees for 18-20 hours. Final washes were in 0.5x SSC, 0.1% SDS at 65 degrees. Positive clones were identified after autoradiography.

### Heavy chain genomic library construction

To isolate the variable region gene for the 412 heavy chain, a genomic library was constructed in the lambda vector EMBL-3. High molecular weight DNA was partially digested with restriction endonuclease Sau3A and size-fractioned on a 10-40% sucrose density gradient. DNA fragments of approximately 15-23kb were ligated with EMBL-3 arms and packaged into phage particle in vitro using Gigapack Gold. This library was screened at a density of 30,000 plaques per 150 mm plate using a ^{J}H probe. Hybridization and wash conditions were identical to those used for the light chain library.

### DNA probes

The mouse heavy chain ^{J}H probe is a 2 kb BamHI-/EcoRI fragment containing both J3 and J4 segments. The mouse light chain ^{J}K probe is a 2.7 kb HindIII fragment containing all five ^{J}K segments. ³²P--labeled probes were prepared by random priming using a kit obtained from Boehringer Mannheim. Free nucleotides were removed by centrifugation through a Sephadex G-50 column. The specific activities of the probes were approximately 10⁹ cmp/µg.

### Northern analysis

Ten (10) µg total cellular RNA was subjected to electrophoresis on 1% agarose/formaldehyde gels (Maniatis, et al., Molecular Cloning) and transferred to nitrocellulose. Blots were hybridized with random primed DNA probes in 50% formamide, 2x Denhardt's solution, 5x SSC, and 200 µg/ml denatured salmon sperm DNA at 42 degrees for 10 hours. Final wash conditions were 0.5x SSC 0.1% SDS at 65 degrees.

### DNA transfection using electroporation

Plasmid DNA to be transfected was purified by centrifuging to equilibrium in ethidium bromide/cesium chloride gradients two (2) times. Ten (10)-50 µg of plasmid DNA was added to 10⁷ SP2/0 cells in Hanks salts medium and the mixture placed in a Biorad electroporation apparatus. Electroporation was at 200 volts and the cells were plated out in 96 well microtiter plates. Appropriate drug selection was applied after 48 hours and drug resistant colonies were identified after 1-2 weeks.

### Quantitation of antibody production

Tissue culture supernatant was analyzed for IgG protein content by Elisa assay using standard curves generated with purified IgG. Concentration of chimeric 412 antibody with human constant regions was determined using goat anti-human IgG Fc antibody-coated microtiter plates and alkaline phosphatase conjugated goat anti-human IgG Fc or goat anti-human IgG (H+L) antibody.

Tissue culture supernatant was loaded onto a protein A-sepharose column. The chimeric antibody was eluted from the protein A column with a sodium citrate pH gradient from pH 6.5 to pH 3.5. The purified antibody was concentrated using a Diaflo YM100 ultrafiltration membrane. Antibody concentration was measured by determining the absorbance at 280 nm.

### Indirect Cell Binding Assay on CEM Cells

All samples and standards were diluted to 100 µg/ml with 0.3% gelatin-PBS-0.2% azide. Gelatin-PBS-azide was added to each well of a 96 well microtiter plate; the first column of the plate was left empty An aliquot (150 µl) of each sample and standard was added in duplicate to the (empty) first column of the 96 well plate. Twelve serial 1:4 dilutions were performed by transferring 40 ul each time.

¹²⁵I goat anti-human F(ab')2 was diluted to approximately 300,000 cpm/100 µl (5-10 uCi/µg) in gelatin-PBS-azide.

CEM cells were centrifuged at 1000 rpm, 15 min, the supernatant was discarded and the pellet was resuspended with Hanks buffered saline. Cells were washed twice and a cell count was performed with Trypan Blue. Cells were plated at 7 x 10⁵/well in a V-bottom 96 well polyvinyl plate. To obtain an even distribution of cells, the cell suspension was poured into a petri plate and the plate was swirled gently with one hand while pipetting with the other hand. The plate was centrifuged at 1500 rpm for 5 min and the supernatant was aspirated.

Aliquots (100 µl) of the antibody dilutions were added to each well. The pellet was resuspended by gently pipetting up and down. Cells were incubated for 3 hr at 4°C, then centrifuged at 1500 rpm for 5 min, and the supernatant was aspirated and the pellet was resuspended in 200 µl gelatin-PBS-azide. A spin-wash was performed twice. An aliquot (100 µl) of the 125-I goat anti-mouse F(ab')2 was added to each well. The pellet was resuspended and incubated for 2 hr at room temperature. Cells were spin washed twice and the supernatant was aspirated off. Each well was counted in a gamma counter. The bound counts per minute (cmp) were plotted on the ordinate, antibody concentration was plotted on the abscissa and the concentration at half maximal binding was determined.

### Results

### Cloning of the CD4-specific variable gene regions

Several positive clones were isolated from the heavy and light chain libraries after screening approximately one million plaques using the J_{H} and J_{K} probes, respectively Following at least three (3) rounds of plaque purification, bacteriophage DNA was isolated for each positive clone, digested with either EcoRI (heavy chain clones) or HindIII (light chain clones) and fractionated on 1% agarose gels. The DNA was transferred to nitrocellulose and the blots were hybridized with J_{H} (heavy chain) or J_{K} (light chain) ³²P-labeled DNA probes. For the heavy chain, several clones were obtained that contained 5.5 kb Eco RI DNA fragments that hybridized to the J_{H} probe. The J_{K} probe hybridized to a 5.4 kb fragment present in several light chain clones.

Cloned DNA corresponding to the authentic heavy and light chain variable regions from the 412 hybridoma should hybridize to mRNA isolated from the hybridoma. Non-functional DNA rearrangements at either the heavy or light chain loci should not be expressed. The subcloned fragments were labeled with ³²P by random priming and hybridized to northern blots containing total RNA derived from 653 (the fusion partner of the 412 hybridoma) or from 412. The 5.5 kb EcoRI heavy chain fragment hybridized with a 2 kb EcoRI heavy chain fragment in 412 RNA, but not in 653 RNA. Similarly, the 5.4 kb light chain HindIII fragment hybridized with a 1250 by mRNA in 412 RNA, but not in 653 RNA. These are the correct sizes for heavy and light chain mRNAs respectively. Because the cloned DNA fragments contain sequences expressed in the 412 hybridoma, these data suggest that these are the correct variable region sequences from the 412 hybridoma. The final functional test, however, is the demonstration that these sequences, when combined with the appropriate constant region sequences, are capable of directing the synthesis of an antibody with a specificity and affinity similar to that of the murine 412 antibody.

### Vectors and expression systems

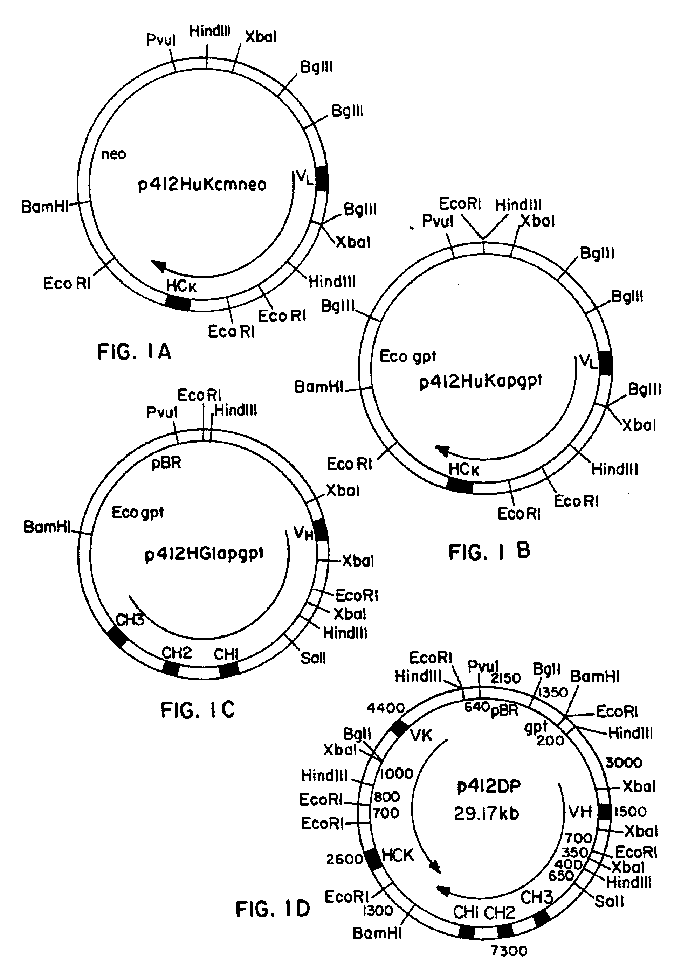

The putative light and heavy chain V genes cloned from the 412 hybridoma were joined to human kappa and G1 constant region genes in expression vectors. The 5.5 kb EcoRI fragment corresponding to the putative heavy chain V region gene from 412 was used to replace the 17-1A V_{H} EcoRI fragment of the previously described vector pSV2ΔHgpt17-1AV_{H}-hCG1 (Sun, L. et al., PNAS 84, p. 214-218 (1987)) to yield p412HG1apgpt. For the light chain, two (2) different constructions were made. In the first construction, the 5.4 kb putative light chain fragment from 412 was used to replace the 17-1 A HindIII fragment of pSV184-ΔHneo17-1 AVKhCK to yield p412HuKcmneo. In the second construction, the 5.4 kb HindIII fragment from 412 was cloned into a similar vector to pSV184ΔHneo17--1AVhCK except that the selectable marker for mammalian cells was Ecogpt instead of neo. The resulting plasmid was designated p412HuKapgpt. A plasmid was also constructed containing both heavy and light chain genes in the same plasmid, and was designated p412-DP. The 412 expression plasmids are shown in figure 1.

To express the chimeric heavy and light chain genes, various combinations of the expression plasmids were transfected into the non-producing mouse myeloma cell line SP2/0. The heavy chain vector was co-transfected with either the neo or gpt version of the light chain vector, and p412-DP was transfected alone. Mycophenolic acid selection was applied after 24 hours; for cotransfections with neo and gpt vectors, selection with both mycophenolic acid and G418 was used. Resistant colonies were expanded to stable cell lines and tissue culture supernatant from these cell lines was tested for antibody using an ELISA assay with goat anti-human IgG Fc antibody and goat anti-human H+L conjugated with alkaline phosphatase (Jackson Laboratories). A cell line designated JL3A3 was chosen for further study

The chimeric 412 antibody was purified from tissue culture supernatant of cell line JL3A3 by Protein A Sepharose chromatography. 190 mls of supernatant was adjusted to 0.1 M Tris, 0.002 M EDTA, pH 8.0 and loaded on a 12 ml Protein A Sepharose column equilibrated in 0.1 M Tris, 0.002 M EDTA, pH 8.0. The column was bashed to baseline, and the IgG was eluted with a pH gradient from 0.1 M citrate pH 6.5 to 0.1 M citrate pH 3.5, with the IgG peak eluting at approximately pH 4.0. The peak was pooled, neutralized with 1 M Tris, diafiltered into PBS, and 0.2 micron filtered. 5.4 mg was recovered (approximately 28 ug/ml starting supernatant).

The IgG was evaluated for purity by HPLC and SDS-PAGE. HPLC analysis on GF-250 gel filtration showed a single peak with an apparent molecular weight of approximately 150,000. SDS-PAGE on Pharmacia Phastgel 10-15% (4 ml samples) visualized with Coomasie stain also showed a clean preparation of the same molecular weight.

The purified IgG was evaluated for immunoactivity in an indirect cell binding assay on CEM cells. CEM cells display the CD4 receptor on their surface. Chimeric 7E3 G1, an irrelevant antibody, was run as a negative control. Relative affinity values were determined from the inverse of the concentration (M) at half maximal binding. The values obtained are as follows:

| Sample | Tracer | Relative Affinity |
|---|---|---|
| cC123B JL3A3 (chimeric 412) | anti human F (ab')₂ | 2.5 x 10⁹ M⁻¹ |
| cC123B JL3A3 (chimeric 412) | anti human Fc | 8.0 x 10⁹ M⁻¹ |
| c123 Clinical Vial (murine 412) | anti mouse F(ab')₂ | 6.5 x 10⁹ M⁻¹ |

The data demonstrate that chimeric anti-CD4 IgG1 binds to CEM cells with an affinity similar to the murine anti-CD4 antibody. This indicates that the murine and chimeric antibodies have similar affinities for the human CD4 receptor.

### Biological Information

To assess safety, pharmacokinetics, and CD4 effects, a preferred chimeric anti-CD4 monoclonal antibody of the invention (cM-T412) was administered intravenously (IV) for seven days at a dose of 5 mg/kg/day to four chimpanzees. The antibody was well tolerated and circulating CD4 cell number was markedly decreased from the first dose through 2-3 weeks after the last dose. CD4 positive cells increased in number 3-4 weeks post dose, but remained depressed in treated animals relative to saline treated controls for 3-4 months. No anti-chimeric antibody response was detected. The prolonged depletion of circulating CD4+ T-cells with no incidence of adverse effects or immunogenic response supports the potential clinical application of such chimeric anti-CD4 monoclonal antibodies to the treatment of autoimmune disease, such as, for example, rheumatoid and psoriatic arthritis, MS, SLE, and myasthenia gravis.

Significant improvement of symptoms was also observed in a series of 15 human patients with refractory rheumatoid arthritis treated with single IV doses ranging from 1-200 mg of a preferred chimeric anti-CD4 antibody of the invention, CT-M412. Signficiant decreases in swollen joints and tender joints were observed up to 21 days and 90 days, respectively, post treatment. The antibody was well tolerated, with only transient flu-like symptoms observed. Modest anti-mouse response occurred in 8/15 patients. Sustained decreases in CD4+ T-cells occurred up to 14 days post treatment, and were present though less pronounced at 35 days. CD8+ ceils were also transiently decreased following treatment, but reverted to baseline by 72 hours. Clinical dosage may vary depending on the nature and severity of the condition being treated, as well as the age and weight of the patient and the existence of concurrent conditions; however, single IV doses in he range of 1-20 mg are expected to be clinically useful.

While binding of M-T412 to both CEM and human T lymphocytes can be blocked by M-T151, there are certain functional differences between these antibodies. In vivo evidence includes a report of clinical trials in rheumatoid arthritis patients in whom M-T151 effected improvement in symptoms with only a transient decrease in circulating CD4+ cells and an increase in CD+8 cells. Herzog et al., J. Autoimmunity 2: 627-642 (1989). In contrast, in vivo administration of the cM-T412 antibody results in long-term depletion of CD4+ T cells as well as a depletion of CD8+ T cells. Although this difference in biological activity between the murine antibody, M-T151, and the chimeric antibody, cM-T412, could result in part from the human Fc portion of the chimeric antibody which may better recruit human effector functions, however, clinical reports with chimeric anti-leu3a monoclonal antibody (which like cM-T412 is of the gamma 1 isotype), and well as evidence from in vitro assays of M-T412 and M-T151 suggest that at least some differences in epitope specificity may be involved. For example, in evaluating the effects of antibodies on the proliferation of peripheral blood mononuclear cells (PBMC) in response to tetanus toxoid, the cM-T412 was more effective than the parent murine M-T412; both were more effective than the M-T151 (murine). Since M-T412 and M-T151 are both of the murine G2a isotype, differences between them are most likely due to differences in the Fab regions. Because of its ability to down regulate both CD4 and CD8 subsets of T cells, the chimeric anti-CD4 monoclonal antibodies of the invention designated cM-T412 is more preferred. cM-T412 has also been observed to down regulate both activate cell surface IL-2 receptors and soluble IL-2.

### Equivalents

Those skilled in the art will recognize, or be able to ascertain using no more than routine experimentation, many equivalents to the specific embodiments of the invention described herein. Such equivalents are intended to be encompassed by the following claims.

## Claims (Claims for the following Contracting State(s): AT, BE, CH, LI, DE, DK, FR, GB, IT, LU, NL, SE)

1. A CD4-specific chimeric immunoglobulin comprising an antigen binding region of non-human origin and a constant region of human origin, the chimeric immunoglobulin being capable of inducing sustained depletion of CD4⁺ T cells in humans and able to compete with the M-T151 mAb for binding to CEM cells.

2. A chimeric immunoglobulin of Claim 1, wherein the antigen binding region is derived from a murine anti-CD4 immunoglobulin.

3. A chimeric immunoglobulin of Claim 2, wherein the antigen binding region is derived from a monoclonal antibody.

4. An antigen binding fragment of a chimeric immunoglobulin of Claim 1, comprising an antigen binding region of non-human origin and at least a portion of a constant region of human origin.

5. A chimeric immunoglobulin comprising:
a. at least one chimeric heavy chain comprising an antigen binding region derived from the heavy chain of a non-human immunoglobulin specific for CD4 receptor linked to at least a portion of a human heavy chain constant region, the heavy chain being in association with:
b. at least one chimeric light chain comprising an antigen binding region derived from a light chain of the non-human immunoglobulin linked to at least a portion of a human light chain constant region, the chimeric immunoglobulin being; (1) capable of inducing sustained depletion of CD4⁺ T cells in humans, and (2) able to compete with the M-T151 mAb for binding to CEM cells.

6. A chimeric immunoglobulin of Claim 5 of the IgG1 isotype, e.g. IgG1 kappa isotype.

7. A chimeric immunoglobulin of Claim 5 or Claim 6, wherein the antigen binding region is derived from a murine antibody.

8. An antigen binding fragment of a chimeric immunoglobulin according to any one of Claims 5 to 7 which is a Fab, Fab' or F(ab')₂ fragment, comprising a murine variable region specific for the CD4 receptor complex and a human constant region.

9. A chimeric immunoglobulin according to any one of Claims 1-3 and 5-7 or a fragment of Claims 4 or 8 which exhibits high affinity binding to CD4, for example having a Ka of at least 10⁸M⁻¹, e.g. at least 10⁹M⁻¹.

10. A fused gene encoding the chimeric immunoglobulin of any one of Claims 1-3, 5-7 and 9 or fragment of Claims 4 or 8.

11. An expression vector containing the fused gene of Claim 10 in expressible form.

12. The chimeric immunoglobulin of any one of Claims 1-3, 5-7 and 9 or fragment of Claims 4 or 8 for use in therapy, for example in the treatment of autoimmune disorders, e.g. rheumatoid and psoriatic arthritis, SLE, multiple sclerosis and myasthenia gravis.

13. Use of the chimeric immunoglobulin of any one of Claims 1-3, 5-7 and 9 or fragment of Claims 4 or 8, the fused gene of Claim 10 or the expression vector of Claim 11 for the manufacture of a medicament for the treatment of autoimmune disorders, e.g. rheumatoid and psoriatic arthritis, SLE, multiple sclerosis and myasthenia gravis.

## Claims (Claims for the following Contracting State(s): GR, ES)

1. A method for producing a CD4-specific chimeric immunoglobulin comprising an antigen binding region of non-human origin and a constant region of human origin, the chimeric immunoglobulin being capable of inducing sustained depletion of CD4⁺ T cells in humans and able to compete with the M-T151 mAb for binding to CEM cells, the method comprising the step of recovering the expressed immunoglobulins or immunoglobulin chain and optionally further comprising the steps of:
(a) cloning DNA segments encoding the heavy and light chain variable regions of a non-human antibody specific for CD4;
(b) joining at least the functional portion of the DNA segments of (a) to respective DNA segments encoding human heavy and light chain-constant regions to produce a chimeric immunoglobulin encoding gene;
(c) expressing the immunoglobulin encoding gene to produce chimeric immunoglobulin.

2. The method of Claim 1, wherein the antigen binding region is derived from a murine anti-CD4 immunoglobulin.

3. The method of Claim 2, wherein the antigen binding region is derived from a monoclonal antibody.

4. The method of Claim 1, 2 or 3, wherein the immunoglobulin produced is so antigen binding fragment of the chimeric immunoglobulin comprising an antigen binding region of non-human origin and at least a portion of a constant region of human origin.

5. A method for producing a chimeric immunoglobulin comprising:
a. at least one chimeric heavy chain comprising an antigen binding region derived from the heavy chain of a non-human immunoglobulin specific for CD4 receptor linked to at least a portion of a human heavy chain constant region, the heavy chain being in association with:
b. at least one chimeric light chain comprising an antigen binding region derived from a light chain of the non-human immunoglobulin linked to at least a portion of a human light chain constant region, the chimeric immunoglobulin being; (1) capable of inducing sustained depletion of CD4⁺ T cells in humans, and (2) able to compete with the M-T151 mAb for binding to CEM cells, the method comprising the step of recovering the expressed immunoglobulin or immunoglobulin chain and optionally further comprising the steps of:
(a) cloning DNA segments encoding the heavy and light chain variable regions of a non-human antibody specific for CD4;
(b) joining at least the functional portion of the DNA segments of (a) to respective DNA segments encoding human heavy and light chain-constant regions to produce a chimeric immunoglobulin encoding gene;
(c) expressing the immunoglobulin encoding gene to produce chimeric immunoglobulin.

6. The method of Claim 5 wherein the chimeric immunoglobulin is of the IgG1 isotype, e.g. IgG1 kappa isotype.

7. The method of Claim 5 or Claim 6 wherein the antigen binding region is derived from a murine antibody.

8. The method of any one of Claims 5 to 7 wherein the chimeric immunoglobulin is an antigen binding fragment which is a Fab, Fab' or F(ab')₂ fragment, comprising a murine variable region specific for the CD4 receptor complex and a human constant region.

9. The method of any one of Claims 1-8 wherein the chimeric immunoglobulin or fragment thereof exhibits high affinity binding to CD4, for example having a Ka of at least 10⁸M⁻¹, e.g. at least 10⁹M⁻¹.

10. A method for producing a fused gene encoding the chimeric immunoglobulin as defined in any one of Claims 1-3, 5-7 and 9 or fragment as defined in Claims 4 or 8, comprising the steps (a) and (b) as defined in Claim 1.

11. The method of producing an expression vector containing the fused gene produced by the method of Claim 10 in expressible form, comprising the steps of producing the fused gene according to the method of Claim 10 and assembling or inserting the fused gene into an expression vector.

12. The chimeric immunoglobulin as defined in any one of Claims 1-3, 5-7 and 9 or fragment as defined in Claims 4 or 8 for use in therapy, for example in the treatment of autoimmune disorders, e.g. rheumatoid and psoriatic arthritis, SLE, multiple sclerosis and myasthenia gravis.

13. Use of the chimeric immunoglobulin as defined in any one of Claims 1-3, 5-7 and 9 or fragment as defined in Claims 4 or 8, the fused gene as defined in Claim 10 or the expression vector as defined in Claim 11 for the manufacture of a medicament for the treatment of autoimmune disorders, e.g. rheumatoid and psoriatic arthritis, SLE, multiple sclerosis and myasthenia gravis.

## Patentansprüche (Patentansprüche für folgende(n) Vertragsstaat(en): AT, BE, CH, LI, DE, DK, FR, GB, IT, LU, NL, SE)

1. Ein CD4-spezifisches schimäres Immunoglobulin, das eine Antigenbindungsregion nichthumanen Ursprungs und eine konstante Region humanen Ursprungs umfaßt, wobei das schimäre Immunoglobulin in der Lage ist, eine dauerhafte Abnahme von CD4⁺ T-Zellen in Menschen zu bewirken und mit dem M-T151 mAb um Bindung an CEM-Zellen konkurrieren kann.

2. Ein schimäres Immunoglobulin nach Anspruch 1, worin die Antigenbindungsregion von einem murinen anti-CD4 Immunoglobulin abgeleitet ist.

3. Ein schimäres Immunoglobulin nach Anspruch 2, worin die Antigenbindungsregion von einem monoklonalen Antikörper abgeleitet ist.

4. Ein Antigen bindendes Fragment eines Schimären Immunoglobulins nach Anspruch 1, das eine Antigenbindungsregion nichthumanen Ursprungs und mindestens einen Teil einer konstanten Region humanen Ursprungs umfaßt.

5. Ein schimäres Immunoglobulin, das umfaßt:
a. mindestens eine schimäre schwere Kette, die eine Antigenbindungsregion umfaßt, die von der schweren Kette eines für den CD4-Rezeptor spezifischen nichthumanenen Immunoglobulins abgeleitet ist und mit mindestens einem Teil der konstanten Region einer humanen schweren Kette verknüpft ist, wobei die schwere Kette assoziiert ist mit:
b. mindestens einer schimären leichten Kette, die eine Antigenbindungsregion umfaßt, die von einer leichten Kette des nichthumanen Immunoglobulins abgeleitet ist und mit mindestens einem Teil der konstanten Region einer humanen leichten Kette verknüpft ist, wobei das schimäre Immunoglobulin (1) in der Lage ist, eine dauerhafte Abnahme von CD4⁺ T-Zellen in Menschen zu bewirken und (2) mit dem M-T151 mAb um Bindung an CEM-Zellen konkurrieren kann.

6. Ein schimäres Immunoglobulin nach Anspruch 5 vom IgG1 Isotyp, z. B. IgG1 kappa Isotyp.

7. Ein schimäres Immunoglobulin nach Anspruch 5 oder Anspruch 6, worin die Antigenbindungsregion von einem murinen Antikörper abgeleitet ist.

8. Ein Antigen bindendes Fragment eines schimären Immunoglobulins gemäß einem der Ansprüche 5 bis 7, das ein Fab, Fab' oder F(ab)₂ Fragment ist, das eine für den CD4-Rezeptorkomplex spezifische murine variable Region und eine humane konstante Region umfaßt.

9. Ein schimäres Immunoglobulin gemäß einem der Ansprüche 1 bis 3 und 5 bis 7 oder ein Fragment nach Anspruch 4 oder 8, das eine Bindung hoher Affinität an CD4 zeigt, zum Beispiel eine Ka von mindestens 10⁸M⁻¹ besitzt, z. B. mindestens 10⁹M⁻¹.

10. Ein fusioniertes Gen, das das schimäre Immunoglobulin nach einem der Ansprüche 1 bis 3, 5 bis 7 und 9 oder Fragment nach Anspruch 4 oder 8 codiert.

11. Ein Expressionsvektor, der das fusionierte Gen nach Anspruch 10 in exprimierbarer Form enthält.

12. Das schimäre Immunoglobulin nach einem der Ansprüche 1 bis 3, 5 bis 7 und 9 oder Fragment nach Anspruch 4 oder 8 für die Verwendung in der Therapie, zum Beispiel bei der Behandlung von Autoimmunkrankheiten, z. B. rheumatoider Arthritis und Arthritis psoriatica, SLE, Multiple Sklerose und Myasthenia gravis.

13. Verwendung des schimären Immunoglobulins nach einem der Ansprüche 1 bis 3, 5 bis 7 und 9 oder Fragments nach Anspruch 4 oder 8, des fusionierten Gens nach Anspruch 10 oder des Expressionsvektors nach Anspruch 11 zur Herstellung eines Medikaments für die Behandlung von Autoimmunkrankheiten, z. B. rheumatoider Arthritis und Arthritis psoriatica, SLE, Multiple Sklerose und Myasthenia gravis.

## Patentansprüche (Patentansprüche für folgende(n) Vertragsstaat(en): GR, ES)

1. Ein Verfahren zur Herstellung eines CD4-spezifischen schimären Immunoglobulins, das eine Antigenbindungsregion nichthumanen Ursprungs und eine konstante Region humanen Ursprungs umfaßt, wobei das schimäre Immunoglobulin in der Lage ist, eine dauerhafte Abnahme von CD4⁺ T-Zellen in Menschen zu bewirken und mit dem M-T151 mAb um Bindung an CEM-Zellen konkurrieren kann, wobei das Verfahren den Schritt der Wiedergewinnung des exprimierten Immunoglobulins oder der exprimierten Immunoglobulin-Kette umfaßt und gegebenenfalls zusätzlich die Schritte umfaßt:
(a) Klonieren von DNS-Abschnitten, die die variablen Regionen der schweren und leichten Kette eines nichthumanen Antikörpers, der spezifisch für CD4 ist, codieren;
(b) Verbinden mindestens des funktionalen Teils der DNS-Abschnitte von (a) mit entsprechenden DNS-Abschnitten, die konstante Regionen der humanen schweren und leichten Kette codieren, um ein Gen herzustellen, das ein schimäres Immunoglobulin codiert;
(c) Exprimieren des Immunoglobulin codierenden Gens, um ein schimäres Immunoglobulin herzustellen.

2. Das Verfahren nach Anspruch 1, worin die Antigenbindungsregion von einem murinen anti-CD4 Immunoglobulin abgeleitet ist.

3. Das Verfahren nach Anspruch 2, worin die Antigenbindungsregion von einem monoklonalen Antikörper abgeleitet ist.

4. Das Verfahren nach Anspruch 1, 2 oder 3, worin das hergestellte Immunoglobulin ein Antigen bindendes Fragment des schimären Immunoglobulins ist, das eine Antigenbindungsregion nichthumanen Ursprungs und mindestens einen Teil einer konstanten Region humanen Ursprungs umfaßt.

5. Ein Verfahren zur Herstellung eines schimären Immunoglobulins, das umfaßt:
a. mindestens eine schimäre schwere Kette, die eine Antigenbindungsregion umfaßt, die von der schweren Kette eines für den CD4-Rezeptor spezifischen nichthumanen Immunoglobulins abgeleitet ist und mit mindestens einem Teil der konstanten Region einer humanen schweren Kette verknüpft ist, wobei die schwere Kette assoziiert ist mit:
b. mindestens einer schimären leichten Kette, die eine Antigenbindungsregion umfaßt, die von einer leichten Kette des nichthumanen Immunoglobulins abgeleitet ist und mit mindestens einem Teil der konstanten Region einer humanen leichten Kette verknüpft ist, wobei das schimäre Immunoglobulin (1) in der Lage ist, eine dauerhafte Abnahme von CD4⁺ T-Zellen in Menschen zu bewirken und (2) mit dem M-T151 mAb um Bindung an CEM-Zellen konkurrieren kann, wobei das Verfahren den Schritt der Wiedergewinnung des exprimierten Immunoglobulins oder der exprimierten Immunoglobulin-Kette umfaßt und gegebenenfalls zusätzlich die Schritte umfaßt:
(a) Klonieren von DNS-Abschnitten, die die variablen Regionen der schweren und leichten Kette eines nichthumanen Antikörpers, der spezifisch für CD4 ist, codieren;
(b) Verbinden mindestens des funktionalen Teils der DNS-Abschnitte von (a) mit entsprechenden DNS-Abschnitten, die konstante Regionen der humanen schweren und leichten Kette codieren, um ein Gen herzustellen, das ein schimäres Immunoglobulin codiert;
(c) Exprimieren des Immunoglobulin codierenden Gens, um ein schimäres Immunoglobulin herzustellen.

6. Das Verfahren nach Anspruch 5, worin das schimäre Immunoglobulin vom IgG1 Isotyp ist, z. B. IgG1 kappa Isotyp.

7. Das Verfahren nach Anspruch 5 oder Anspruch 6, worin die Antigenbindungsregion von einem murinen Antikörper abgeleitet ist.

8. Das Verfahren nach einem der Ansprüche 5 bis 7, worin das schimäre Immunoglobulin ein Antigen bindendes Fragment ist, das ein Fab, Fab' oder F(ab)₂ Fragment ist, das eine für den CD4-Rezeptorkomplex spezifische murine variable Region und eine humane konstante Region umfaßt.

9. Das Verfahren nach einem der Ansprüche 1 bis 8, worin das schimäre Immunoglobulin oder Fragment davon eine Bindung hoher Affinität an CD4 zeigt, zum Beispiel eine Ka von mindestens 10⁸M⁻¹ besitzt, z. B. mindestens 10⁹M⁻¹.

10. Ein Verfahren zur Herstellung eines fusionierten Gens, das das schimäre Immunoglobulin, wie in einem der Ansprüche 1 bis 3, 5 bis 7 und 9 definiert, oder Fragment, wie in Anspruch 4 oder 8 definiert, codiert, und das Verfahren die Schritte (a) und (b), wie in Anspruch 1 definiert, umfaßt.

11. Ein Verfahren zur Herstellung eines Expressionsvektors, der das fusionierte Gen, das mit dem Verfahren nach Anspruch 10 hergestellt wird, in exprimierbarere Form enthält und das Verfahren die Schritte zur Herstellung des fusionierten Gens gemäß dem Verfahren nach Anspruch 10 und Zusammenbau oder Insertion des fusionierten Gens in einen Expressionsvektor umfaßt.

12. Das schimäre Immunoglobulin, wie in einem der Ansprüche 1 bis 3, 5 bis 7 und 9 definiert, oder Fragment, wie in Anspruch 4 oder 8 definiert, für die Verwendung in der Therapie, zum Beispiel bei der Behandlung von Autoimmunkrankheiten, z. B. rheumatoider Arthritis und Arthritis psoriatica, SLE, Multiple Sklerose und Myasthenia gravis.

13. Verwendung des schimären Immunoglobulins, wie in einem der Ansprüche 1 bis 3, 5 bis 7 und 9 definiert, oder Fragments, wie in Anspruch 4 oder 8 definiert, des fusionierten Gens, wie in Anspruch 10 definiert, oder des Expressionsvektors, wie in Anspruch 11 definiert, zur Herstellung eines Medikaments für die Behandlung von Autoimmunkrankheiten, z. B. rheumatoider Arthritis und Arthritis psoriatica, SLE, Multiple Sklerose und Myasthenia gravis.

## Revendications (Revendications pour l'(les) Etat(s) contractant(s) suivant(s): AT, BE, CH, LI, DE, DK, FR, GB, IT, LU, NL, SE :)

1. Immunoglobuline chimère spécifique du CD4, comprenant une région de liaison à l'antigène qui n'est pas d'origine humaine et une région constante d'origine humaine, ladite l'immunoglobuline chimère étant capable d'induire un épuisement prolongé de cellules T CD4⁺ chez les humains et apte à entrer en compétition avec l'anticorps monoclonal M-T151 pour la fixation aux cellules CEM.

2. Immunoglobuline chimère selon la revendication 1, dans laquelle la région de liaison à l'antigène est dérivée d'une immunoglobuline murine anti-CD4.

3. Immunoglobuline chimère selon la revendication 2, dans laquelle la région de liaison à l'antigène est dérivée d'un anticorps monoclonal.

4. Fragment de liaison à l'antigène d'une immunoglobuline chimère selon la revendication 1, comprenant une région de liaison à l'antigène qui n'est pas d'origine humaine et au moins une partie d'une région constante d'origine humaine.

5. Immunoglobuline chimère comprenant
a. au moins une chaîne lourde chimère comprenant une région de liaison à l'antigène dérivée de la chaîne lourde d'une immunoglobuline non humaine spécifique du récepteur CD4 reliée à au moins une partie d'une région constante d'une chaîne lourde humaine, ladite chaîne lourde étant associée à
b. au moins une chaîne légère chimère comprenant une région de liaison à l'antigène dérivée d'une chaîne légère de l'immunoglobuline non humaine reliée à au moins une partie d'une région constante d'une chaîne légère humaine, ladite immunoglobuline chimère étant (1) capable d'induire un épuisement prolongé de cellules T CD4⁺ chez les humains, et (2)apte à entrer en compétition avec l'anticorps monoclonal M-T1 51 pour la fixation aux cellules CEM.

6. Immunoglobuline chimère selon la revendication 5, d'isotype IgG1, par exemple, d'isotype kappa IgG1.

7. Immunoglobuline chimère selon la revendication 5 ou la revendication 6, dans laquelle la région de liaison à l'antigène est dérivée d'un anticorps murin.

8. Fragment de liaison à l'antigène d'une immunoglobuline chimère selon l'une quelconque des revendications 5 à 7 qui est un fragment Fab, Fab' ou F(ab')2, comprenant une région variable murine spécifique du complexe récepteur CD4 et une région constante humaine.

9. Immunoglobuline chimère selon l'une quelconque des revendications 1-3 et 5-7 ou fragment selon les revendications 4 ou 8 présentant une haute affinité de liaison au CD4, ayant, par exemple, un Ka d'au moins 10⁸ M⁻¹, par exemple d'au moins 10⁹ M⁻¹.

10. Gène de fusion codant pour l'immunoglobuline chimère selon l'une quelconque des revendications 1-3, 5-7 et 9 ou pour le fragment selon les revendications 4 ou 8.

11. Vecteur d'expression contenant le gène de fusion selon la revendication 10 sous une forme exprimable.

12. Immunoglobuline chimère selon l'une quelconque des revendications 1-3, 5-7 et 9 ou fragment selon les revendications 4 ou 8 pour utilisation en thérapie, par exemple dans le traitement de maladies auto-immunes, par exemple, de l'arthrite rhumatoïde et psoriatique, du SLE, de la sclérose en plaques et de la myasténie grave.

13. Utilisation de l'immunoglobuline chimère selon l'une quelconque des revendications 1-3, 5-7 et 9, ou d'un fragment selon la revendication 4 ou 8, ou du gène de fusion selon la revendication 10, ou du vecteur d'expression selon la revendication 11, pour la préparation d'un médicament pour le traitement de maladies auto-immunes, par exemple, de l'arthrite rhumatoïde et psoriatique, du SLE, de la sclérose en plaques et de la myasténie grave.

## Revendications (Revendications pour l'(les) Etat(s) contractant(s) suivant(s): GR, ES)

1. Procédé de préparation d'une immunoglobuline chimère spécifique du CD4, comprenant une région de liaison à l'antigène qui n'est pas d'origine humaine et une région constante d'origine humaine, ladite immunoglobuline chimère étant capable d'induire un épuisement prolongé de cellules T CD4⁺ chez les humains et apte à entrer en compétition avec l'anticorps monoclonal M-T151 pour la fixation aux cellules CEM, le procédé comprenant l'étape consistant à récupérer les immunoglobulines ou la chaîne immunoglobulinique exprimées et comprenant éventuellement en outre les étapes consistant à :
(a) cloner des segments d'ADN codant pour les régions variables de chaînes lourde et légère d'un anticorps non humain spécifique de CD4 ;
(b) relier au moins la partie fonctionnelle des segments d'ADN de (a) aux segments d'ADN respectifs codant pour les régions constantes de chaînes lourdes et légères humaines afin de produire un gène codant pour l'immunoglobuline chimère ;
(c) exprimer le gène codant pour l'immunoglobuline afin de produire une immunoglobuline chimère.

2. Procédé selon la revendication 1, dans lequel la région de liaison à l'antigène est dérivée d'une immunoglobuline murine anti-CD4.

3. Procédé selon la revendication 2, dans lequel la région de liaison à l'antigène est dérivée d'un anticorps monoclonal.

4. Procédé selon la revendication 1, 2 ou 3, dans lequel l'immunoglobuline produite est un fragment de liaison à l'antigène de l'immunoglobuline chimère comprenant une région de liaison à l'antigène qui n'est pas d'origine humaine et au moins une partie d'une région constante d'origine humaine.

5. Procédé de préparation d'une immunoglobuline chimère comprenant :
a. au moins une chaîne lourde chimère comprenant une région de liaison à l'antigène dérivée de la chaîne lourde d'une immunoglobuline non humaine spécifique du récepteur CD4 reliée à au moins une partie d'une région constante d'une chaîne lourde humaine, ladite chaîne lourde étant associée à
b. au moins une chaîne légère chimère comprenant une région de liaison à l'antigène dérivée d'une chaîne légère de l'immunoglobuline non humaine reliée à au moins une partie d'une région constante d'une chaîne légère humaine, ladite immunoglobuline chimère étant (1) capable d'induire un épuisement prolongé de cellules T CD4⁺ chez les humains et (2) apte à entrer en compétition avec l'anticorps monoclonal M-T151 pour la fixation aux cellules CEM, le procédé comprenant l'étape consistant à récupérer les immunoglobulines ou la chaîne immunoglobulinique exprimées et comprenant éventuellement en outre les étapes consistant à :
(a) cloner des segments d'ADN codant pour les régions variables de chaînes lourde et légère d'un anticorps non humain spécifique de CD4 ;
(b) relier au moins la partie fonctionnelle des segments d'ADN de (a) aux segments d'ADN respectifs codant pour les régions constantes de chaînes lourdes et légères humaines pour produire un gène codant pour l'immunoglobuline chimère ;
(c) exprimer le gène codant pour l'immunoglobuline afin de produire une immunoglobuline chimère.

6. Procédé selon la revendication 5, dans lequel l'immunoglobuline chimère est d'isotype IgG1, par exemple, d'isotype kappa IgG1.

7. Procédé selon la revendication 5 ou la revendication 6, dans lequel la région de liaison à l'antigène est dérivés d'un anticorps murin.

8. Procédé selon l'une quelconque des revendications 5 à 7, dans lequel l'immunoglobuline chimère est un fragment de liaison qui est un fragment Fab, Fab' ou F(ab')2, comprenant une région variable murine spécifique du complexe récepteur CD4 et une région constante humaine.

9. Procédé selon l'une quelconque des revendications 1-8, dans lequel l'immunoglobuline chimère ou son fragment présente une haute affinité de liaison au CD4, ayant, par exemple, un Ka d'au moins 10⁸M⁻¹, par exemple d'au moins 10⁹ M⁻¹.

10. Procédé de préparation d'un gène de fusion codant pour l'immunoglobuline chimère telle que définie dans l'une quelconque des revendications 1-3, 5-7 et 9 ou pour un fragment tel que défini dans les revendications 4 ou 8, comprenant les étapes (a) et (b) telles que définies à la revendication 1.

11. Procédé de préparation d'un vecteur d'expression contenant le gène fusionné produit par le procédé de la revendication 10 sous une forme exprimable, comprenant les étapes consistant à produire le gène fusionné selon le procédé de la revendication 10 et à assembler ou à insérer le gène fusionné dans un vecteur d'expression.

12. Immunoglobuline chimère telle que définie dans l'une quelconque des revendications 1-3, 5-7 et 9 ou fragment tel que défini dans les revendications 4 ou 8 pour utilisation en thérapie, par exemple dans le traitement de maladies auto-immunes, par exemple, de l'arthrite rhumatoïde et psoriatique, du SLE, de la sclérose en plaques et de la myasténie grave.

13. Utilisation de l'immunoglobuline chimère telle que définie dans l'une quelconque des revendications 1-3, 5-7 et 9, ou d'un fragment tel que défini dans la revendication 4 ou 8, ou du gène de fusion tel que défini dans la revendication 10, ou du vecteur d'expression tel que défini la revendication 11, pour la préparation d'un médicament pour le traitement de maladies auto-immunes, par exemple, de l'arthrite rhumatoïde et psoriatique, du SLE, de la sclérose en plaques et de la myasténie grave.
